(19) **Europäisches Patentamt**
**European Patent Office**
**Office européen des brevets**

(11) **EP 4 722 721 A1**

(12) **EUROPEAN PATENT APPLICATION**
published in accordance with Art. 153(4) EPC

(43) Date of publication:
08.04.2026 Bulletin 2026/15

(51) International Patent Classification (IPC):
*G01N 33/543* *(2006.01)*

(21) Application number: 23939465.3

(52) Cooperative Patent Classification (CPC):
G01N 33/543

(22) Date of filing: 26.05.2023

(86) International application number:
PCT/JP2023/019667

(87) International publication number:
WO 2024/246980 (05.12.2024 Gazette 2024/49)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
KH MA MD TN

(71) Applicant: KOWA COMPANY, LTD.
Aichi 460-8625 (JP)

(72) Inventor: HIRONO, Taisuke
Tokyo 103-8433 (JP)

(74) Representative: Ribeiro, James Michael
Withers & Rogers LLP
2 London Bridge
London SE1 9RA (GB)

(54) **INSPECTION DEVICE, INSPECTION SYSTEM, INSPECTION METHOD, AND STORAGE MEDIUM**

(57) A test apparatus is provided. The test apparatus includes a data acquisition device that acquires image data including: a color-developing section that reacts with a target substance in a specimen to develop color; and a reference section that displays a sample color used to determine color of the color-developing section and a reference color used to correct positioning-induced variations; and a determination device that determines an amount of the target substance based on color information of the color-developing section, color information of the sample color, and color information of the reference color, which are acquired from the image data.

FIG. 4

**Description**

TECHNICAL FIELD

The present invention relates to a test apparatus, a test system, a test method, and a storage medium.

BACKGROUND

Test devices utilizing immunochromatography methods are disclosed as devices for performing antibody tests, antigen tests, and the like (see, for example, patent documents 1 and 2). For example, the test devices described in patent documents 1 and 2 encapsulate a test strip within a case with a specimen application window and a detection window that allows for visual observation of the state of the test strip, wherein labeled antigen or antibody and capture antigen or antibody are immobilized on the test strip. Specifically, the test strip is provided with labeled antigen or labeled antibody immobilized at the position of the specimen application window and capture antigen or antibody immobilized in a line at the position of the detection window. The labeled antigen or labeled antibody is labeled, for example, with metal colloid. When a specimen is applied to the application window, the antibody or antigen in the specimen forms a complex with the labeled antigen or labeled antibody. The complex migrates toward the detection window of the test strip and is captured by the capture antigen or antibody. This capture process leads to the development of color on the test strip at the fixing position of the labeled antigen, etc., resulting in a line appearing at the detection window. The presence of this line indicates whether the specimen contains the antibody or antigen.

The level of the antibody etc. can also be determined based on the color tone of the line by comparing it with a determination card with an array of color samples. One approach to performing the determination using the aforementioned test devices automatically rather than visually is to perform image analysis on the lines. However, the color tones in the images may differ from the actual color tones depending on the imaging skills and imaging environments during the image capture (such as lighting conditions during the imaging and imaging circumstances). Therefore, depending on the imaging environments and other factors, the color tones of the lines in the image may vary significantly, potentially leading to variations in test results.

In one disclosed technology, facial image data of a patient captured at the patient's terminal is transmitted to a medical institution, and various medical diagnoses are performed based on the facial image data (patent document 3). Technologies that analyze image data acquired by imaging a specimen alongside a predetermined color sample or color grid are also known in the art (patent documents 4 and 5). However, the technologies in patent documents 3 to 5 do not address variations in determinations caused by differences in the color tones of captured images due to variations in imaging skills and environments.

PRIOR ART LITERATURES

Patent Literatures

[Patent Document 1] JP 2022-119435 A
[Patent Document 2] JP 2016-102790 A
[Patent Document 3] JP H10-165375 A
[Patent Document 4] JP 2016-161301 A
[Patent Document 5] JP 2017-515106 A

SUMMARY OF INVENTION

In light of the aforementioned issues, an objective of the present invention is to provide a test apparatus, test system, test method, and storage medium for appropriately determining the amount of a target substance in a specimen.

According to one embodiment, the test apparatus includes: a data acquisition device that acquires image data including a color-developing section that reacts with a target substance in a specimen to develop color and a reference section displaying a sample color used for determining the color of the color-developing section and a reference color used for correcting positioning-induced variations; and a determination device that determines an amount of the target substance based on color information of the color-developing section, color information of the sample color, and color information of the reference color, which are acquired from the image data.

According to one embodiment, a test system includes: a data acquisition device that acquires image data including a color-developing section that reacts with a target substance in a specimen to develop color and a reference section displaying a sample color used to determine the color of the color-developing section and a reference color used to correct positioning-induced variations; and a determination device that determines an amount of the target substance based on color information of the color-developing section, color information of the sample color, and color information of the reference color, which are acquired from the image data.

According to one embodiment, a test method includes acquiring image data that includes a color-developing section that reacts with a target substance in a specimen to develop color and a reference section displaying a sample color used to determine the color of the color-developing section and a reference color used to correct positioning-induced variations; and determining an amount of the target substance based on color information of the color-developing section, color information of the sample color, and color information of the reference color, which are acquired from the image data.

According to one embodiment, a non-transitory storage medium that stores a program causes a control device to: acquire image data that includes a color-developing section that reacts with a target substance in a specimen to develop color and a reference section displaying a sample color used to determine the color of the color-developing section and a reference color used to correct positioning-induced variations; and determine an amount of the target substance based on color information of the color-developing section, color information of the sample color, and color information of the reference color, which are acquired from the image data.

BRIEF DESCRIPTION OF THE DRAWINGS

FIG. 1 is a block diagram showing an outline of a test system, according to one embodiment.
FIG. 2 is a block diagram showing the configuration of a user terminal, according to one embodiment.
FIG. 3 is a block diagram showing the configuration of a test apparatus, according to one embodiment.
FIG. 4 is a front view showing an example configuration of a test kit.
FIG. 5 is a front view showing a test device of the test kit.
FIG. 6 is a front view showing a base sheet of the test kit.
FIG. 7 is an illustration showing an example of image data.
FIG. 8 is a flowchart showing an automatic determination process that occurs during the operation of a test system.
FIG. 9 shows the relationship between the estimated saturation value of color information at the position of a first color-developing section and the amount of a target substance.
FIG. 10 is a flowchart showing the operation of a test system.
FIG. 11 is a front view showing an example modification of a test kit or image data.
FIG. 12 is a front view showing an example modification of a test kit or image data.
FIG. 13 is a front view showing an example modification of a test kit or image data.
FIG. 14 is a front view showing an example modification of a test kit or image data.

DETAILED DESCRIPTION

[Embodiment]

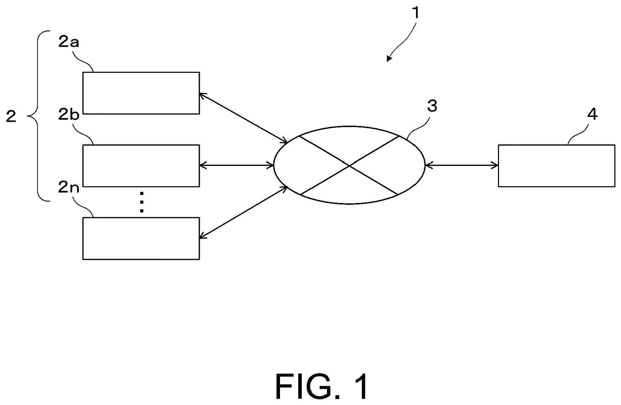

In the following, a description is given of a test system, etc., according to one embodiment, with reference to the drawings.
Referring to FIGS. 1 to 3, an example configuration of a test system 1 is described. FIG. 1 is a block diagram showing an outline of the test system 1. The test system of this embodiment is configured to determine the amount of a target substance in a specimen by acquiring image data of a test device placed on a predetermined base sheet and performing image processing on the image data to correct the color information of the image data.
As shown in FIG. 1, the test system 1 includes a test apparatus 4. The test apparatus 4 is connected to user terminals 2 (2a, 2b, ..., 2n) via communication lines 3.
Alternatively, the test system 1 may be considered as including the test apparatus 4 and the user terminals 2.

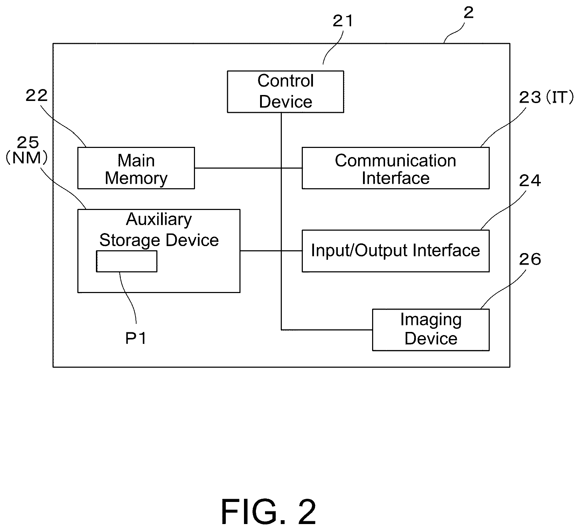

FIG. 2 is a block diagram showing the configuration of a user terminal 2. The user terminal 2 is, for example, an information terminal operated by the user. The user terminal 2 images a test device 5 (described later) in test and transmits the image data from the user terminal 2 to the test apparatus 4. The user is a person who provides the image data for testing to the test apparatus 4. The user may be the examinee who provides the specimen, or may be a different person from the examinee.
Examples of user terminal 2 include various computers such as personal computers, smartphones, and tablet devices.
The user terminal 2 includes a control device 21, a main memory 22, a communication interface 23, an input/output interface 24, an auxiliary storage device 25, and an imaging device 26.
The control device 21 includes an arithmetic processing device such as a CPU, retrieves computer programs P1 and data stored in the auxiliary storage device 25, and executes predetermined information processing (specifically, various processes executed at the user terminal 2). The main memory 22 includes a memory device such as a DRAM, and temporarily stores data necessary for the control device 21 to execute arithmetic processing.
The communication interface 23 is an interface for communicating with the test apparatus 4. The communication interface 23, which functions as a data transmission device IT, transmits image data acquired by the imaging device 26 to the test apparatus 4. The communication interface 23 also transmits various data necessary for testing to the test apparatus 4. The communication interface 23 also receives test result data from the test apparatus 4.
The input/output interface 24 is an interface for receiving input signals from devices such as a keyboards, mice, and touch panels and for outputting various output signals to displays and other devices.
The auxiliary storage device 25 is a non-volatile storage medium or a non-transitory storage medium NM, such as a hard disk, a flash memory or the like. The auxiliary storage device 25 stores a computer program P1 and data for the control device 21 to execute various information processing. The auxiliary storage device 25 stores data necessary for testing and test result data received from the test apparatus 4, which will be described later. The computer program P1 and the aforementioned data may be stored on a storage medium other than the auxiliary storage device 25 to be provided. Examples of the storage media include flexible disks, hard disks, CD-ROMs, MO (magnetic disks), DVD-ROMs, memory cards, and similar devices. Alternatively, the computer program P1 may be transmitted to the user terminal 2 via the communication line 3 for installation.

Examples of data required for testing include image data, vaccination history, PCR test history, antibody test history, antigen test history, and negative certificate issuance history. Examples of test result data include the amount of the target substance.

The imaging device 26, such as a camera, captures images of the test device 5 and other items as described later to acquire image data.

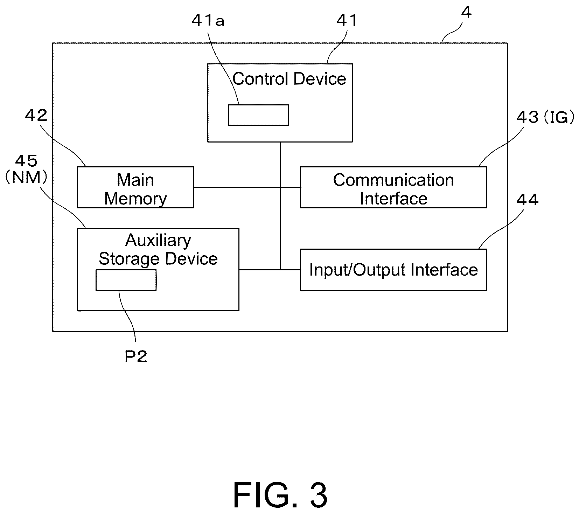

FIG. 3 is a block diagram showing the configuration of the test apparatus 4. The test apparatus 4 may be, for example, an information terminal of a test agency that performs a test based on image data of the test device 5 acquired from the user terminal 2. The test apparatus 4 performs an automatic test, an automatic determination, and the like based on the image data acquired from the user terminal 2, and transmits the test results to the user terminal 2.

The test apparatus 4 may be a general-purpose computer or a multi-computer system comprising multiple computers. Alternatively, the test apparatus 4 may be configured to utilize cloud computing.

The test apparatus 4 includes a control device 41, a main memory 42, a communication interface 43, an input/output interface 44, and an auxiliary storage device 45.

The control device 41 includes an arithmetic processing device, such as a CPU, retrieves a computer program P2 and data stored in the auxiliary storage device 45, and executes predetermined information processing (specifically, various processes described later that are executed by the test apparatus 4). The main memory 42 includes a memory device, such as a DRAM, and temporarily stores data necessary for the control device 41 to execute arithmetic processing.

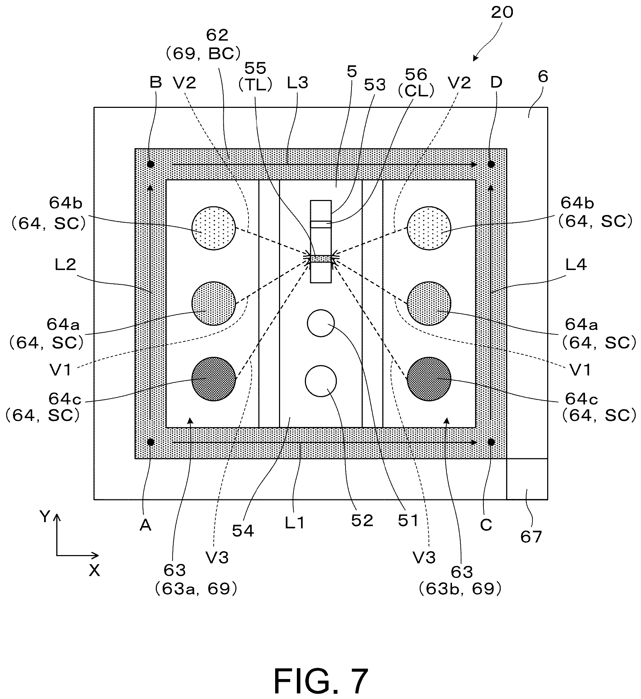

The control device 41 includes a determination device 41a that analyzes the image data acquired from the user terminal 2 and outputs the test results. The control device 41 implements the determination device 41a by retrieving and executing the computer program P2. The determination device 41a determines the amount of the target substance in the specimen based on color information of a first color-developing section 55, color information of sample colors SC, and color information a reference color BC (see Figure 7) which are acquired from image data 20 described later. Specifically, the determination device 41a determines the amount of the target substance by estimating corrected color information for at least one of the first color-developing section 55 and color samples 64 using gradient information calculated from the color information of a reference display section 62 acquired from the image data 20. The operation of the determination device 41a will be described later.

The communication interface 43 is an interface for communicating with the user terminal 2, for example. The communication interface 43, which functions as a data acquisition device IG, receives image data of the test device 5 etc. from the user terminal 2. The communication interface 43 also receives various data necessary for testing from the user terminal 2. The communication interface 43 also transmits test result data to the user terminal 2.

The input/output interface 44 is an interface for receiving input signals from devices such as a keyboards and mice, as well as for outputting various output signals to displays and other devices.

The auxiliary storage device 45, which is a non-volatile storage medium or non-temporary storage medium NM, such as a hard disk or flash memory, stores a computer program P2 and data for the control device 41 to execute various information processing described later. The auxiliary storage device 45 stores the necessary data for testing received from the user terminal 2, as well as test result data. The computer program P2 and the aforementioned data may be stored on a storage medium other than the auxiliary storage device 45 to be provided. Examples of storage media include flexible disks, hard disks, CD-ROMs, MO (magnetic disks), DVD-ROMs, memory cards, and similar devices. Alternatively, the computer program P2 may be transmitted to the test apparatus 4 via the communication line 3 for installation.

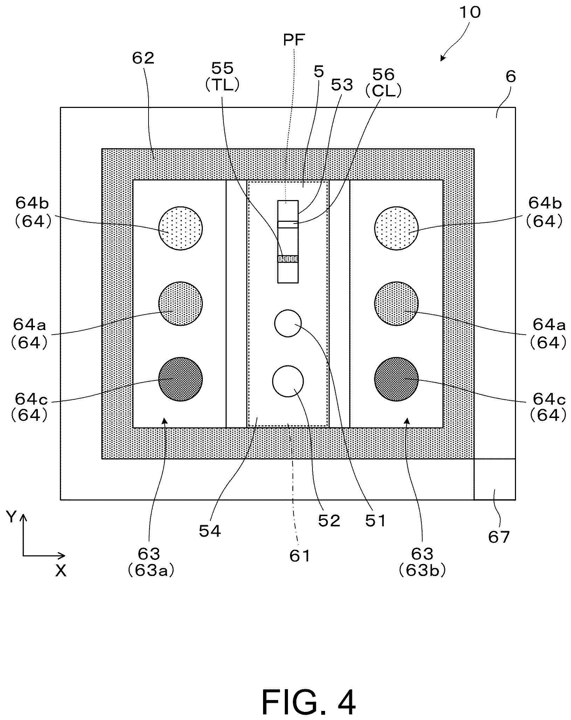

Referring to FIGS. 4 to 6, test kits and other equipment used for testing are described below. While this embodiment uses neutralizing antibody testing as an example, the test target for the test system 1 of this embodiment are not limited to this.

FIG. 4 is a front view showing an example configuration of a test kit 10. The test kit 10 includes a test device 5 and a base sheet 6 on which the test device 5 is placed. The test device 5 and the base sheet 6 are test instruments provided in advance to the test subject by the testing institution.

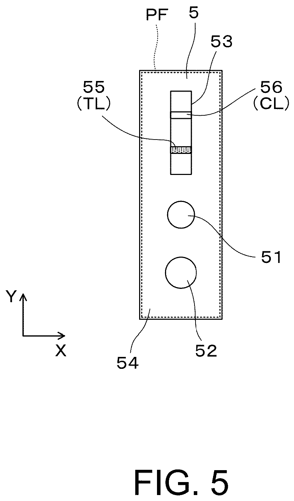

FIG. 5 is a front view of the test device 5 of the test kit 10. The test device 5 has a specimen receiving portion 51, a buffer receiving portion 52, and a result display portion 53. The specimen receiving portion 51, the buffer receiving portion 52, and the result display portion 53 are connected via filter paper PF housed within a cassette portion 54 of the test device 5. The cassette portion 54 exposes the filter paper PF in the areas corresponding to the specimen receiving portion 51, the buffer receiving portion 52, and the result display portion 53.

The specimen receiving portion 51 is a portion for receiving the specimen. The specimen is the material to be tested, for example, blood for a neutralizing antibody test. The specimen may vary depending on the test.

Other than blood, specimens may include saliva, nasopharyngeal swab fluid, nasal swab fluid, urine, tear fluid, other secretions, or a portion of tissue. The specimen contains a target substance to be tested. The target substance is, for example, an antibody, an antigen, etc. Labeled substance that reacts with the target substance to form a complex is immobilized on the filter paper portion corresponding to the specimen receiving portion 51. The labeled substance may be, for example, labeled antigen or labeled antibody. At the specimen receiving portion 51, the target substance and the labeled substance bind to form complex substance, which then moves toward the result display portion 53. The labeled substance may be immobilized on the filter paper portion corresponding to the result display portion 53 instead.

The buffer receiving portion 52 is a portion for receiving buffer solution. The buffer dropped onto the buffer receiving portion 52 moves toward the result display portion 53, assisting the movement of the complex substance.

The result display portion 53 displays the test results regarding the presence or absence of the target substance in the specimen and/or the target substance amount. The filter paper portion corresponding to the result display portion 53 is provided with a capture line TL and a control line CL. On the capture line TL, located upstream, capture substance that specifically binds to the complex substance is adsorbed or immobilized in a line. The capture substance may be, for example, capture antigen, capture antibody, etc. On the control line CL, located downstream, control substance that specifically binds to the labeled substance is immobilized in a line. The control substance may be, for example, control antigen, control antibody, etc.

In the result display portion 53, the binding of the complex substance and the capture substance creates a state where colored particles derived from the labeled substance are concentrated, causing the capture line TL to develop color. This results in a band-shaped first color-developing section 55 appearing on the capture line TL. Additionally, in the result display portion 53, the binding of the labeled substance and the control substance creates a state where colored particles derived from the labeled substance are concentrated, causing the control line CL to develop color. This results in a band-shaped second color-developing section 56 appearing on the control line CL. The second color-developing section 56 develops color when the buffer is properly dropped. If both the first color-developing section 55 and the second color-developing section 56 develop color, the specimen is determined as containing the target substance. Furthermore, the target substance amount is determined based on the color tone (e.g., color intensity) of the first color-developing section 55. The amount of the target substance may be, for example, the antibody titer for a neutralization antibody test.

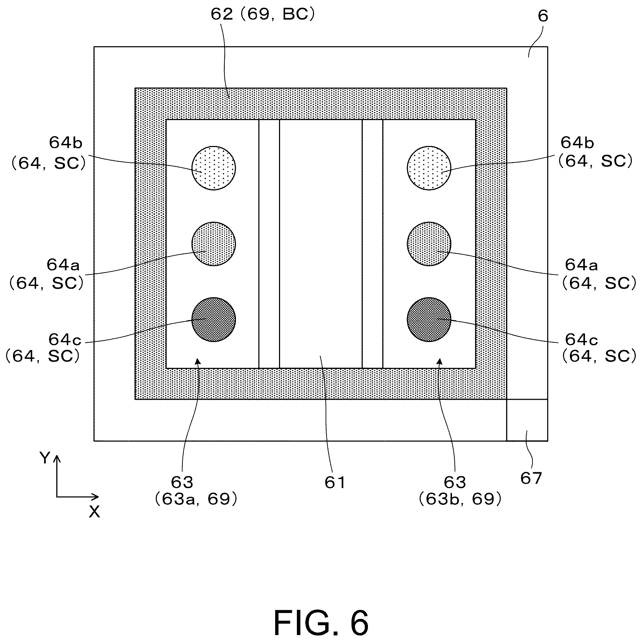

FIG. 6 is a front view showing the base sheet 6 of the test kit 10. FIG. 6 shows the base sheet 6 without the test device 5 placed thereon. The base sheet 6 includes a placement area 61, a reference display section 62, and chart sections 63. The reference display section 62 and the chart sections 63 constitute a reference section 69, which is used for image data processing as described later. The reference section 69 displays sample colors SC used to determine the color of the first color-developing section 55, and the reference color BC used to correct positioning-induced variations. The sample colors SC are the displayed colors of the color samples 64 in the chart sections 63, which will be described later. The reference color BC is the displayed color of the reference display section 62, which will be described later.

The placement area 61 is where the test device 5 is placed. The placement area 61 is positioned approximately at the center of the base sheet 6.

The reference display section 62, which is used for calibration, provides the reference for calculating correction amounts that compensate for illumination-induced color unevenness resulting from imaging skills and environments (such as lighting conditions and imaging situations during image capture). The reference display section 62 is arranged to surround the placement area 61 and the chart sections 63. The reference display section 62 displays the reference color BC for estimating corrected color information acquired by compensating for positioning-induced variations for the first color-developing section 55 and/or the color samples 64 of the chart sections 63. Since a gradient occurs in the image data 20 due to the above imaging environment, etc., the control device 41 of the test apparatus 4, which functions as the determination device 41a, compensates for positioning-induced variations based on gradient information of the reference color BC to estimate corrected color information for the first color-developing section 55 or color samples 64 assuming that the first color-developing section 55 or color samples 64 are located at the positions of the color samples 64 or first color-developing section 55. The reference display section 62 is displayed, for example, in a single reference color BC. Specifically, the reference display section 62 is colored with a color corresponding to predetermined color information (e.g., color components, such as red, green, and blue in the RGB color space, or hue, saturation, and value in the HSV (HSB) color space). Color information is directly acquired from the image data 20. The color information constitutes the image data 20 or the camera image, including hue, saturation, value, etc. Corrected color information is color information that enables comparison by canceling out the effects of the gradient caused by the difference in the positioning of the first color-developing section 55 and the chart sections 63 (or the color samples 64).

The chart sections 63 serve as an indicator to determine the amount of the target substance based on comparison to the color or color information of the first color-developing section 55. The chart sections 63 are arranged at two locations in the two lateral directions, (i.e., the X-directions) relative to the placement area 61.

Specifically, the chart sections 63 include a first chart section 63a on the left and a second chart section 63b on the right. The chart sections 63 are provided with color samples 64 that display the sample colors SC, which are to be compared with the color of the first color-developing section 55. The first chart section 63a and the second chart section 63b are each provided with multiple color samples 64 with different gradations.

The color samples 64 correspond to amounts of the target substance. The color samples 64 carry predetermined color information (e.g., color components, such as red, green, and blue in the RGB color space, or hue, saturation, and value in the HSV (HSB) color space). The color samples 64 are arranged with line symmetry in the first chart section 63a and the second chart section 63b. In other words, the color samples 64 exhibit identical colors and arrangements in the first chart section 63a and the second chart section 63b.

In the shown example, the color samples 64 include first color samples 64a, second color samples 64b, and third color samples 64c. The first color samples 64a, the second color samples 64b, and the third color samples 64c have different gradations one another. For example, the saturation of the first color samples 64a is higher than that of the second color samples 64b and lower than that of the third color samples 64c. In other words, the colors become progressively darker in the order of the second color samples 64b (light color), the first color samples 64a (intermediate color), and third color samples 64c (dark color). Here, the color of the reference display section 62 (the reference color) is the same as the intermediate color out of the multiple color samples 64. The color of the first color samples 64a is the same as that of the reference display section 62, but not limited to this. Additionally, the number and positions of the respective color samples 64 are not limited to what is shown.

The base sheet 6 preferably includes an identification section 67 that carries identification information (such as lot information or serial number information) about the test device 5 and can be readable by any reader terminal (including smartphones and computers). The identification section 67 may be, for example, a two-dimensional barcode. However, the configuration of the identification section 67 is not limited to this. The identification section 67 may be provided on the test device 5.

Using the identification section 67 ensures that the test device 5 displayed in image data 20 (described later) is used for the first time in the current test. This prevents, for example, the reuse of image data 20 from a test device 5 that previously yielded a positive result (indicating the presence of antibodies).

Using the test kit 10 or test device 5 described above simplifies the process of acquiring test results and makes it possible to visually determine the amount of the target substance based on the color tone of the first color-developing section 55.

Referring to FIG. 7, a description is given below of the image data retrieved from the user terminal. FIG. 7 is a diagram showing an example of the image data 20.

The image data 20 is acquired by imaging the base sheet 6 with the test device 5 placed thereon. In other words, the image data 20 is an image containing the test device 5 and the base sheet 6. Specifically, the image data 20 includes the first color-developing section 55, the reference display section 62, and the chart sections 63 of the test device 5. In other words, the image data 20 includes the first color-developing section 55 and the reference section 69.

The color information (or color components) of the pixels displayed in the image data 20 is usually based on the RGB color space. In this embodiment, it is preferable that the control device 41 of the test apparatus 4 converts the RGB color components of each pixel in the acquired image data 20 into color information (hue, saturation, and value) in the HSV (or HSB) color space, which is easier to analyze. However, the control device 41 may not perform color information conversion. In implementations where conversion is performed, the control device 41 may convert the color information to that of a different color space. The control device 41 may convert the color information of all pixels in the image data 20, or convert the color information only of pixels located at specific locations.

When both the first color-developing section 55 and the second color-developing section 56 of the result display portion 53 develop color in the image data 20, the specimen is determined as containing the target substance. Furthermore, the amount of the target substance is determined based on the color tone of the first color-developing section 55. The color tone refers to the intensity of the color, which may be indicated, for example, by the level of saturation. When only the second color-developing section 56 develops color, the specimen is determined as not containing the target substance. When only the first color-developing section 55 develops color, it is determined that a test error has occurred.

The color intensity of the first color-developing section 55 varies depending on the amount of the target substance. For example, the saturation of the color information may vary in the first color-developing section 55. The presence of a light color in the first color-developing section 55 indicates a low amount of the target substance. Conversely, the presence of a dark color in the first color-developing section 55 indicates a high amount of the target substance.

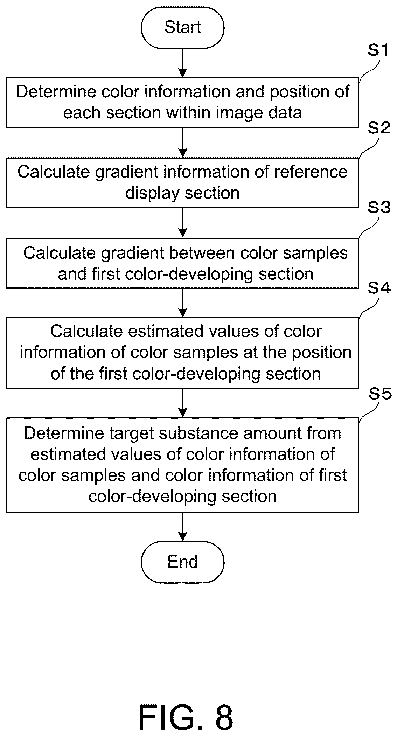

Referring to FIG. 8 and others, a description is given below of automatic determination using the image data in the test apparatus 4. The automatic determination refers to the process of determining the amount of the target substance in the specimen based on the color determination of the first color-developing section 55. FIG. 8 is a flowchart showing the automatic determination process within the operation of the test system 1.

As described above, the color tone of the first color-developing section 55 serves as an indicator to determine the amount of the target substance in the test device 5. When the test uses the image data 20 of the test device 5 captured by the user terminal 2, the color tone of the first color-developing section 55 displayed in the acquired image may differ from its actual color tone due to variations in the imaging skills and environments. The imaging skills and environments differ for each user. Therefore, significant variations in the color tone of the first color-developing section 55 within the image data 20 are anticipated. Furthermore, the illuminance distribution of the illumination light that illuminates the test device 5 and the reflected light distribution from the test device 5 vary depending on the test device 5. Therefore, the color tone (color information) of the first color-developing section 55 displayed in the image data 20 may be influenced not only by the imaging angle but also by the relative position of the test device 5 with respect to the illumination.

Furthermore, the illuminance distribution of the light that illuminates the base sheet 6 and the reflected light distribution from the base sheet 6 also vary depending on the location of the base sheet 6. Consequently, the color information of the reference display section 62 and the chart sections 63 (the color samples 64) displayed in the image data 20 may also differ from the actual color information.

In light of the above, the control device 41 of the test apparatus 4 performs correction processing on the image data 20 based on the correction values from the reference display section 62, thereby correcting the variations in color information for the first color-developing section 55 and the chart sections 63.

In advance, the control device 41 of the test apparatus 4, which operates as the determination device 41a, identifies the color information and position of each section within the image data 20 acquired from the user terminal 2 (step S1). In implementations where determination of the presence or absence of the target substance (referred to as the determination during testing) is performed as described later, this identification is preferably performed after acquiring the image data 20 but before the determination during testing.

The control device 41 of the test apparatus 4, which operates as the determination device 41a, calculates the gradient information of the reference display section 62 within the image data 20 (step S2). Here, the gradient information refers to the amount of change in color information per unit pixel or per unit length. Specifically, the control device 41 calculates the gradient information of the color information (e.g., saturation) corresponding to the reference display section 62 from one position to another within the reference display section 62. For example, the control device 41 calculates gradient information L1 (gradient information along the X-direction) from point A to point C within the reference display section 62 shown in FIG. 7, and gradient information L2 (gradient information along the Y-direction) from point A to point B within the reference display section 62. The method for calculating the gradient information may be changed as appropriate.

Subsequently, the control device 41 of the test apparatus 4, which operates as the determination device 41a, calculates the gradients between the color samples 64 and the first color-developing section 55 (step S3). Here, the gradients refer to the amounts of changes in color information corresponding to the color samples 64 between the color samples 64 and the first color-developing section 55. The gradients are the differential correction values in specific directions, corresponding to the correction values of the color samples 64. Specifically, the control device 41 calculates the gradient V1 of the color information (saturation) corresponding to the first color samples 64a between the first color samples 64a and the first color-developing section 55 based on the gradient information L1, the gradient information L2, the positions of the first color samples 64a, and the position of the first color-developing section 55. This allows, for example, the gradient V1 in the directions from the first color-developing section 55 toward the first color samples 64a as shown in FIG. 7, to be inferred.

For example, when the gradient information is denoted as L1, the gradient information as L2, the position (coordinates) of the first color sample 64a as (Xa, Ya), and the position (coordinates) of the first color-developing section 55 as (Xs, Ys), the gradient V1 is calculated using the following equation:

$$V1 = (Xs - Xa) \times L1 + (Ys - Ya) \times L2,$$

where the value $(Xs - Xa) \times L1$ represents the gradient of color information along the X direction, and the value $(Ys - Ya) \times L2$ represents the gradient of color information along the Y direction.

The control device 41 of the test apparatus 4, which operates as the determination device 41a, calculates estimated values of the color information (or corrected color information) of the color samples 64 at the position of the first color-developing section 55 (step S4). Specifically, the control device 41 estimates the corrected color information of the first color samples 64a at the position of the first color-developing section 55 from the gradient V1. When the color information of the first color samples 64a is denoted as Cs, the corrected color information Ca of the first color samples 64a at the position of the first color-developing section 55 is calculated using the following expression. In step S5 described later, the estimated values calculated for the respective paired first color samples 64a are averaged. Similar calculations may be performed for color samples 64 at other positions; however, it is preferable to unify the signs (positive or negative) of the values of the color information.

$$Ca = Cs + V1.$$

For example, consider the case where the following is derived from the image data 20: gradient information L1 = 1; gradient information L2 = 2; the position of the first color sample 64a (Xa, Ya) = (20, 20); and the position of the first color-developing section 55 (Xs, Ys) = (30, 25). In this scenario, when the color information Cs (saturation) of the first color sample 64a is 40, the corrected color information Ca of the first color sample 64a at the position of the first color-developing section 55 is calculated as 60 using the above expression. Specifically, the gradient of color information along the X-direction from X=20 to X=30 is (Xs - Xa) × L1 = (30 - 20) × 1 = 10. The gradient of color information along the Y direction from Y = 20 to Y = 25 is (Ys - Ya) × L2 = (25 - 20) × 2 = 10. Therefore, the gradient V1 of the color information corresponding to the first color sample 64a between the first color sample 64a and the first color-developing section 55 is 20. Accordingly, the correction color information Ca for the first color sample 64a at the position of the first color-developing section 55 is estimated as Cs + V1 = 40 + 20 = 60. It should be noted that the calculation methods for the gradient and the estimated values of color information are mere examples and may be modified as appropriate.

The control device 41 of the test apparatus 4, which operates as the determination device 41a, performs the same function for the second color samples 64b and the third color samples 64c; the control device 41 determines the color information gradients V2 and V3 corresponding to the second color samples 64b (or third color samples 64c) between the second color samples 64b (or the third color samples 64c) and the first color-developing section 55, and estimates the corrected color information of the second color samples 64b (or the third color samples 64c) at the position of the first color-developing section 55.

Subsequently, the control device 41 of the test apparatus 4, which operates as the determination device 41a, determines the amount of the target substance based on the estimated values of the color information (corrected color information) of the color samples 64 and the color information of the first color-developing section 55 (step S5). Specifically, the control device 41 determines the amount of the target substance corresponding to the first color-developing section 55 based on the estimated value of the color information (estimated saturation) of each color sample 64 at the position of the first color-developing section 55 and the color information (saturation) of the first color-developing section 55.

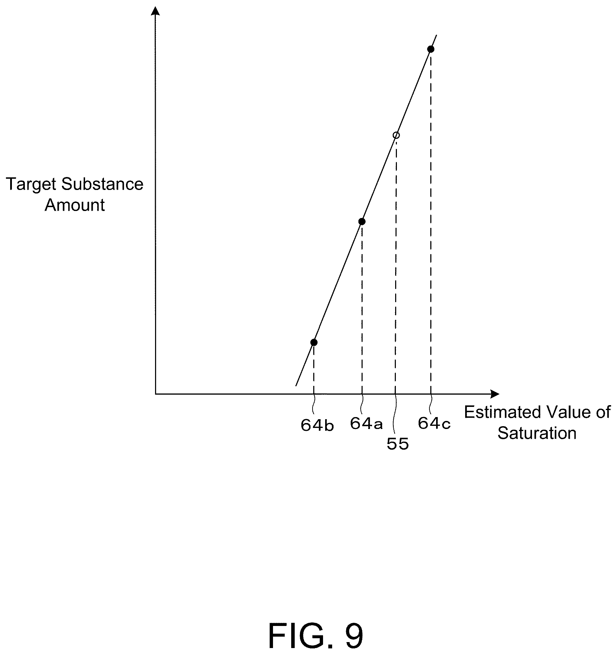

FIG. 9 is a diagram showing the relationship between the amount of the target substance and the estimated saturation value out of the correction color information at the position of the first color-developing section 55. The horizontal axis of FIG. 9 represents the estimated saturation value at the position of the first color-developing section 55 acquired based on the estimated saturation values of the respective color samples 64a to 64c calculated in step S4. The vertical axis of FIG. 9 represents the antibody titer, which is an example of the amount of the target substance. The first color samples 64a correspond, for example, to an antibody titer of 50 BAU/mL. The second color samples 64b correspond, for example, to an antibody titer of 15 BAU/mL. The third color samples 64c correspond, for example, to an antibody titer of 100 BAU/mL.

The amount of the target substance is determined, for example, by adopting the estimated value of the saturation of the color sample 64 whose saturation is close to that of the first color-developing section 55. The amount of the target substance is preferably determined under the conditions that the saturation of the first color-developing section 55 falls between the estimated saturation values of two different color samples 64.

For example, consider the case where the color information (saturation) of the first color-developing section 55 is 70. In step S4, the corrected color information (saturation) of the first color samples 64a at the position of the first color-developing section 55 is estimated to be 60, and the corrected color information (saturation) of the third color samples 64c is estimated to be 80. Therefore, antibody titer determination is based on the change from the estimated value of the saturation of the first color samples 64a to the estimated value of the saturation of the third color sample 64c. When the color information (saturation) of the first color-developing section 55 is 70, the antibody titer increases by 2.5 for each increase of 1 in saturation. Meanwhile, the difference between the saturation of the first color samples 64a at the position of the first color-developing section 55 and the saturation of the first color-developing section 55 itself is 10. Accordingly, when the antibody titer corresponding to the first color samples 64a is 50 BAU/mL, the antibody titer corresponding to the color information of the first color-developing section 55 can be calculated as 50 + 2.5 × 10 = 75 BAU/mL. Thus, the antibody titer determined from the color information of the first color-developing section 55 is 75 BAU/mL.

It should be noted that the number of the estimated values of the color information (corrected color information) determined for the color samples 64 may be one or more than one. In other words, if the change in the amount of the target substance against the saturation is known, a single estimated value may suffice. Furthermore, even if the saturation of the first color-developing section 55 lies outside the calculated estimated values, the amount of the target substance can be determined by predicting the aforementioned change.

While the estimated values are calculated for saturation among the color information in the above, estimated values may be calculated for value or hue instead. Furthermore, estimated values may be calculated for a combination of saturation, value, and hue.

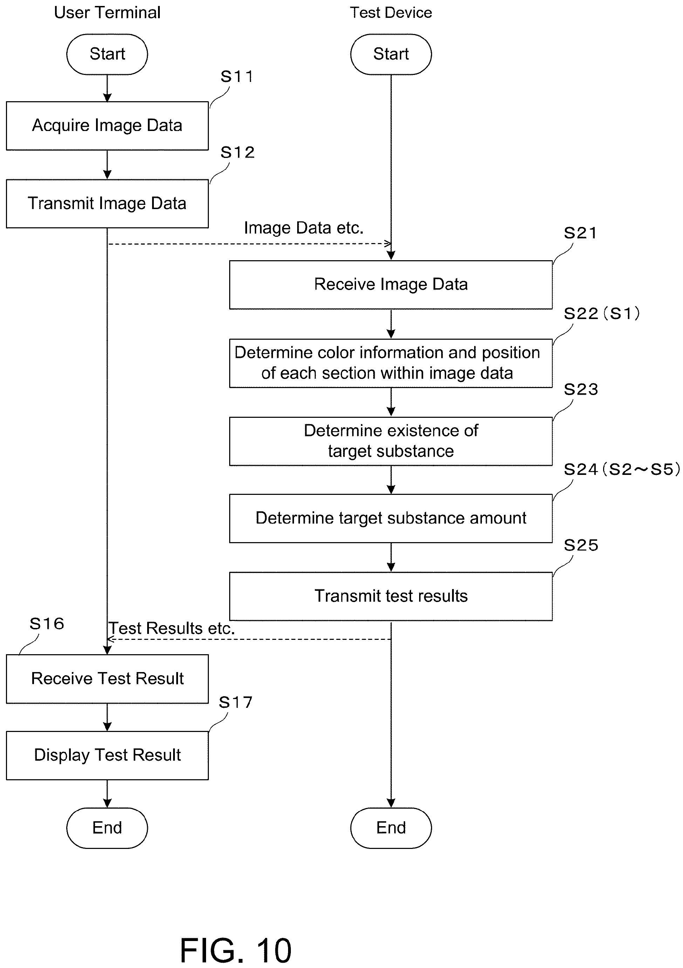

Referring to FIG. 10, the operation of the test system according to one embodiment is described below. FIG. 10 is a flowchart showing the operation of the test system 1.

First, the control device 21 of the user terminal 2 controls the operation of the imaging device 26 to acquire image data 20 (step S11). Specifically, the control device 21 controls the operation of the imaging device 26 to capture an image of the test device 5, where the first color-developing section 55 and the second color-developing section 56 appear in the result display portion 53, and the base sheet 6 on which the test device 5 is placed on the placement area 61. This yields image data 20 containing the test device 5, the reference display section 62, and the chart sections 63.

Subsequently, the control device 21 of the user terminal 2 transmits the image data 20 to the test apparatus 4 via the communication interface 23 (step S12). At this moment, the control device 41 also transmits necessary data for testing to the test apparatus 4.

Subsequently, the control device 41 of the test apparatus 4 receives or acquires the image data 20 from the user terminal 2 via the communication interface 43 (step S21).

Subsequently, the control device 41 of the test apparatus 4, which operates as the determination device 41a, identifies the color information and position of each section within the image data 20 (step S22). Specifically, the control device 41 identifies the color information and position of the first color-developing section 55, the color information and position of the reference display section 62, and the color information and positions of the chart sections 63 (each color sample 64), respectively, which are displayed in the image data 20.

Subsequently, the control device 41 of the test apparatus 4, which operates as the determination device 41a, determines the presence or absence of the target substance in the specimen (step S23). Specifically, the control device 41 determines that the specimen contains the target substance when both the first color-developing section 55 and the second color-developing section 56 of the result display portion 53 develop color in the image data 20. When only the second color-developing section 56 develops color, the control device 41 determines that the specimen does not contain the target substance. When only the first color-developing section 55 develops color, the control device 41 determines that a test error has occurred.

Subsequently, the control device 41 of the inspection device 4, which operates as the determination device 41a, determines the amount of the target substance in the specimen (step S24). In step S24, the above-described processes in steps S2 to S5 are performed to determine the amount of the target substance based on various information acquired for the first color-developing section 55, the reference display section 62, and the chart sections 63 (e.g., color information and positional information for each section).

Subsequently, the control device 41 of the test apparatus 4 transmits the test results to the user terminal 2 via the communication interface 43 (step S25). The test results include information regarding the presence or absence of the target substance and the amount of the target substance, for example. This allows the user to confirm the amount of the target substance contained in the specimen, etc. The control device 41 stores the test results along with the necessary data for the test in the auxiliary storage device 45.

Subsequently, the control device 21 of the user terminal 2 receives the test results from the test apparatus 4 via the communication interface 23 (step S16). The control device 21 stores the test results in the auxiliary storage device 25.

After step S16, the control device 21 of the user terminal 2 displays the test results on a display or similar device via the input/output interface 24 (step S17).

As described above, according to one embodiment, the test apparatus 4 can determine the color of the first color-developing section 55 based on the sample colors SC, with the determination reflecting the result of correcting positioning-induced variations by acquiring the image data 20 that includes the reference section 69 displaying both the sample colors SC and the reference color BC along with the first color-developing section 55. The test apparatus 4 corrects the color information of the image data 20 by estimating corrected color information that compensates for positioning-induced variations for the first color-developing section 55 and/or the color samples 64, based on the color information of the reference display section 62. Specifically, the determination device 41a estimates the corrected color information of the color samples 64 in the chart sections 63 at the position of the first color-developing section 55 from the gradient information L1 and L2 of the reference display section 62. The amount of the target substance corresponding to the color information of the first color-developing section 55 is determined based on this corrected color information. As a result, the amount of the target substance in the specimen can be appropriately determined without being affected by the user's imaging skills or the imaging environment.

While the present invention has been described specifically based on the embodiments, the present invention is not limited to these embodiments and may be modified variously without departing from its essence. Furthermore, the various features described in the embodiments may be freely combined as long as there are no technical discrepancies.

The present invention is not limited to the above embodiments and encompasses the following variations. For example, in the above embodiments, the control device 41 of the test apparatus 4, which operates as a determination device 41a, estimates the corrected color information for each color sample 64 at the position of the first color-developing section 55 referring to the color information of the first color-developing section 55 based on the gradient information L1 and L2 acquired from the color information of the reference display section 62. However, the control device 41 may estimate the correction color information of the first color-developing section 55 at the position of each color sample 64 based on the gradient information L1 and L2. In this case, the control device 41 determines the amount of the target substance based on the estimated correction color information for the first color-developing section 55 and the color information of each color sample 64. Alternatively, the control device 41 may estimate the correction color information at a specific position for both the first color-developing section 55 and each color sample 64 to determine the amount of the target substance.

While the control device 41 calculates two gradient information values L1 and L2 from the color information of the reference display section 62 in the above embodiments, the control device 41 may alternatively calculate gradient information at other positions, such as gradient information L3 and L4 shown in FIG. 7. Specifically, the control device 41 may calculate the gradient information L3 (gradient information along the X direction) from point B to point D within the reference display section 62, and the gradient information L4 (gradient information along the Y direction) from point C to point D within the reference display section 62. In this case, more detailed change information regarding the color information of the reference display section 62 can be acquired based on the gradient information L1 to L4. As a result, the control device 41 can more accurately estimate, for example, the corrected color information for each color sample 64 at the position of the first color-developing section 55. The control device 41 may estimate the corrected color information using one of the gradient information L1 to L4. Furthermore, the range for calculating the gradient information may be divided into multiple sections. For example, when calculating gradient information along the X-direction, the control device 41 may divide the calculation range within the reference display section 62 into two sections at an intermediate point between point A and point C. In other words, the control device 41 may calculate the gradient information from point A to the aforementioned intermediate point and the gradient information from the aforementioned intermediate point to point C.

Alternatively, the control device 41 may calculate gradient information along a line that connects any positions within the reference display section 62 or a pair of color samples 64, crossing over the test device 5, the chart sections 63, etc. In this case, the control device 41 can calculate gradient information within the area enclosed by the reference display section 62. Specifically, the control device 41 may calculate gradient information from point A to point D within the reference display section 62 as one example of diagonal gradient information. The control device 41 may calculate, as another example of diagonal gradient information, gradient information along the line connecting the midpoint between points A and B within the reference display section 62 and the midpoint between points B and D. The control device 41 may calculate gradient information along the line connecting the midpoint between points A and B within the reference display section 62 and the midpoint between points C and D as one example of gradient information along the X direction. The control device 41 may calculate gradient information along the line connecting the midpoint between points A and C within the reference display section 62 and the midpoint between points B and D as one example of gradient information along the Y direction. In the above, the calculation range for gradient information may be appropriately modified or combined as necessary.

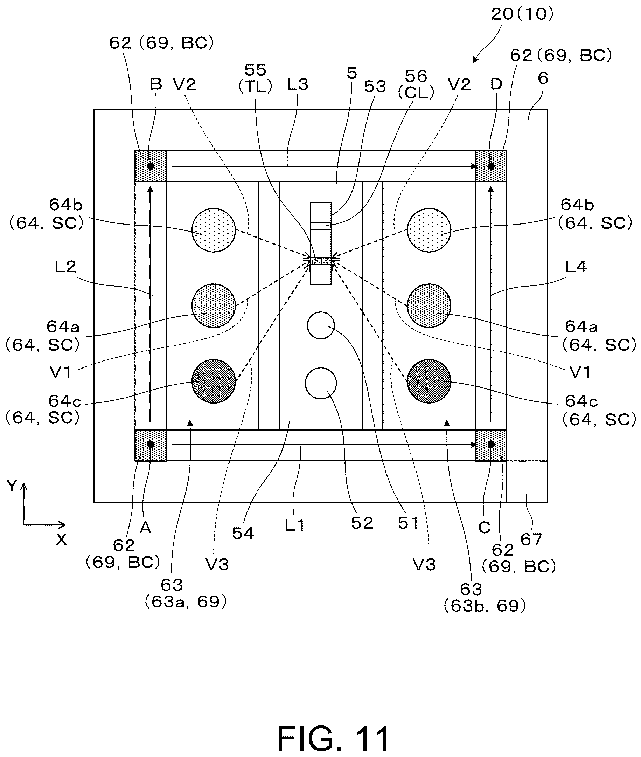

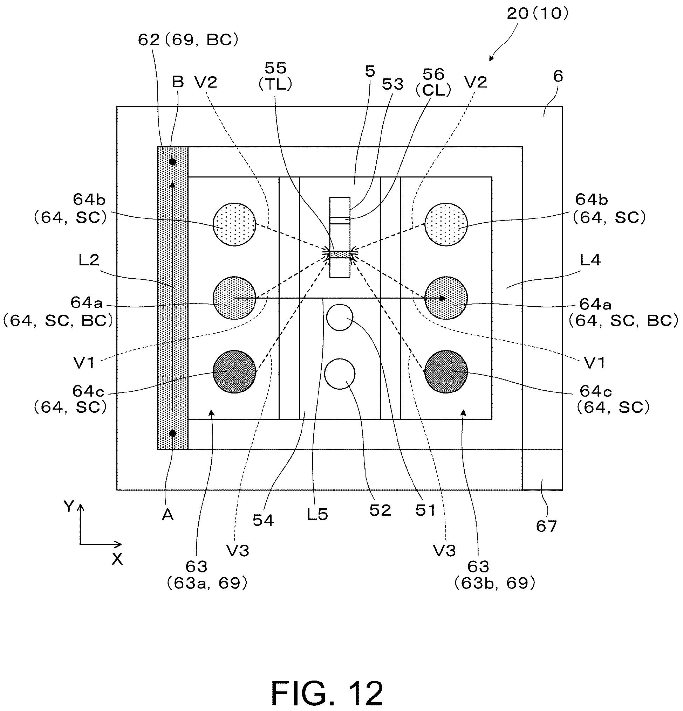

While the reference display section 62 has a frame-like shape surrounding the test device 5 and the chart sections 63 in the above embodiments, the shape of the reference display section 62 is not limited to this. As shown in FIG. 11, for example, reference display sections 62 may be arranged discretely at the four corners of a frame surrounding the test device 5 and the chart sections 63. When the chart sections 63 also serve the role of the reference display section 62 as shown in FIG. 12, the reference display section 62 may, for example, have a strip-like shape extending in the vertical direction (Y-direction) within the image data 20.

In the above embodiments, the control device 41 calculates gradient information L1 and L2 from the color information of the reference display section 62. However, the chart sections 63 may also serve as the reference display section 62 through their arrangement and coloring settings. In this case, the control device 41 calculates gradient information from the color information of the chart sections 63. For example, the control device 41 calculates the Y-direction gradient information L2 from the color information of the reference display section 62 shown in FIG. 12, while calculating the X-direction gradient information L5 from the color information of the two first color samples 64a in the chart sections 63.

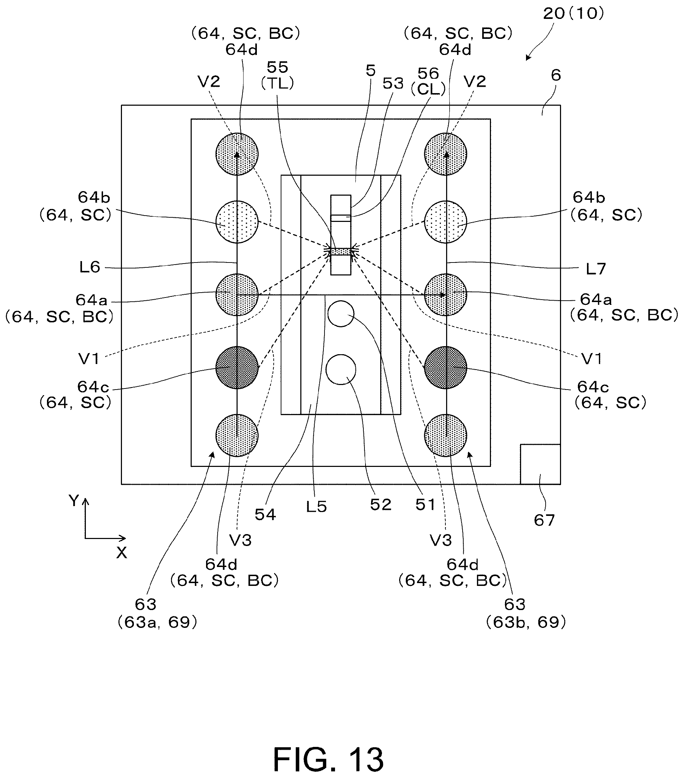

In the above embodiment, the base sheet 6 may not necessarily include the reference display section 62. As shown in FIG. 13, for example, the base sheet 6 may instead include chart sections 63 positioned above, below, to the left, and to the right of the placement area 61, which serve the same function as the reference display section 62. The pair of vertically-arranged chart sections 63 and the pair of horizontally-arranged chart sections 63 each share the same color information. In this case, for example, the control device 41 calculates the Y-direction gradient information L6 and L7 using the two fourth color samples 64d in the upper and lower chart sections 63, and calculates the X-direction gradient information L5 using the two first color samples 64a in the left and right chart sections 63. It is preferable that the first color samples 64a and the fourth color samples 64d have the same color information, but they need not have the same color information. Furthermore, any pair of color samples in the first chart section 63a and the second chart section 63b may be used as the upper and lower fourth color samples 64d. Furthermore, the control device 41 may calculate gradient information using another pair of color samples 64.

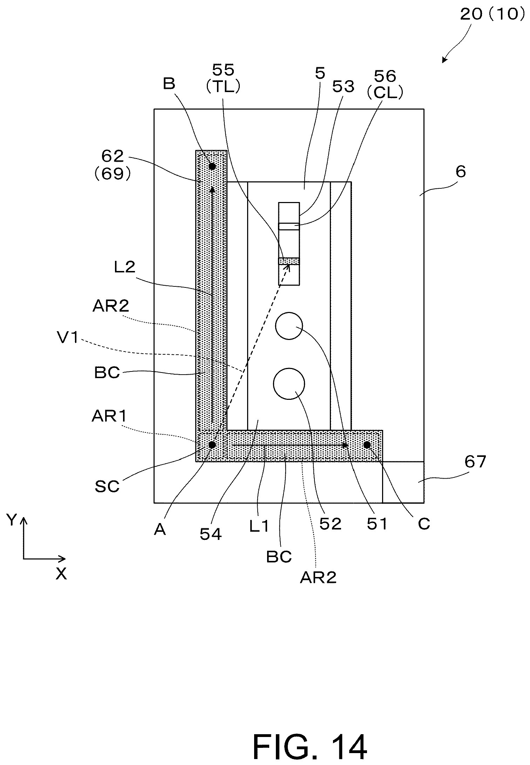

In the above embodiments, the base sheet 6 may not necessarily include the chart sections 63. As shown in FIG. 14, the reference display section 62, serving as the reference section 69, displays the sample colors SC and the reference color BC. In FIG. 14, the area AR1 representing the sample color SC and the area AR2 representing the reference color BC are mere examples and may be changed as appropriate.

With the modified example shown in FIG. 14, the control device 41 can estimate the corrected color information for the sample color SC at the position corresponding to the first color-developing section 55 based on gradient information derived from the reference color BC of the reference display section 62.

Furthermore, in another modified example of FIG. 14, the control device 41 may convert the sample color SC using a conversion formula for standard conditions assuming standard lighting to reflect the sample color SC in the color information of the first color-developing section 55. Specifically, the control device 41 calculates the color information of the corrected sample color by performing gradient correction on the color information of the sample color SC based on the color information of the reference color BC. Subsequently, the control device 41 restores the color of the first color-developing section 55 from the corrected sample color. Here, the color information for the sample color SC and the reference color BC is converted to describe, for example, the stimulus values of the three primary colors and brightness as elements. The stimulus values of the three primary colors and brightness are expressed, for example, in the XYZ (Yxy) color space (where x and y correspond to red and green, respectively). The control device 41 determines a conversion formula that provides a deviation from the standard illumination color under standard conditions for the color information of the corrected sample color. Subsequently, the control device 41 restores the standard illumination color of the first color-developing section 55 by applying the inverse conversion formula of the above conversion formula to the color information of the first color-developing section 55. This enables the control device 41 to estimate the color information of the first color-developing section 55 under the standard conditions. The amount of the target substance in the specimen is calculated based on the restored standard illumination color of the first color-developing section 55, using the relationship formula stored in the auxiliary storage device 45 between the color information of the standard illumination color of the first color-developing section 55 and the amount of the target substance. In this modified example, the color of the first color-developing section 55 can be accurately reproduced using the XYZ color space or the like, and numerical information corresponding to the hue, saturation, and value can be acquired, although the hue, saturation, and value themselves cannot be acquired. This allows the color-developing state of the first color-developing section 55 to be accurately grasped and determined.

In the above embodiments, the gradient may change curvilinearly rather than linearly.

While the control device 41 determines the amount of the target substance to be a specific amount in the above embodiments, the control device 41 may determine an amount range of the target substance. Additionally, the control device 41 may determine the amount of the target substance as a level.

In the above embodiments, elements corresponding to the reference display section 62 and chart sections 63 may be disposed on the cassette portion 54 of the test device 5. In this case, the test kit 10 may not necessarily include the base sheet 6.

The configuration of the test device 5 may be appropriately modified in the above embodiments. For example, the test device 5 may not necessarily include the buffer receiving portion 52. In this case, the mixture of the specimen and the buffer is dispensed onto the specimen receiving portion 51.

Examples of test devices 5 include antibody test devices, antigen test devices, and biomarker test devices. For example, for an antigen test that detects an antigen as the target substance in the specimen, the test device 5, which is used as an antigen test device, may not necessarily include a buffer receiving portion 52. The color intensity of the first color-developing section 55 of the test device 5 changes depending on the amount of antigen contained in the specimen. For antigen testing, similar to antibody testing, the test device 5 is placed on the placement area 61 of the base sheet 6, which includes the chart sections 63 and the reference display section 62, and image data 20 of the test device 5 is acquired with the test device 5 placed on the base sheet 6. Subsequently, the image data 20 is analyzed to determine the amount of the antigen in the specimen based on the color information of the first color-developing section 55 of the test device 5, the color information of the chart sections 63, and the color information of the reference display section 62. For biomarker testing that involves testing a biomarker as a target substance within the specimen, the test device 5, which functions as a biomarker test device, exhibits changes in the color intensity in the first color-developing section 55 of the test device 5 in response to the amount of the biomarker contained within the specimen. For the biomarker testing, similar to antibody testing, image data 20 of the test device 5 is acquired with the test device 5 placed on the base sheet 6, and the amount of biomarker in the specimen is then determined based on the color information of the first color-developing section 55, the color information of the chart sections 63, and the color information of the reference display section 62.

In the above embodiments, the test device 5 and the base sheet 6, particularly the reference display section 62 and the chart sections 63, are preferably formed from materials with low reflectivity.

In the above embodiments, two or more capture lines TL may be provided, and thereby two or more first color-developing sections 55 may appear. In this case, the two or more first color-developing sections 55 may be the same color or different colors.

In the above embodiments, the shapes of the first color-developing section 55 and the second color-developing section 56 are not limited to a line shape. For example, the shapes of the first color-developing section 55 and the second color-developing section 56 may be circular or an array of dots.

In the above embodiments, the test device 5 may employ other immunochromatography methods, such as a vertical flow type, not limited to a lateral flow type that moves the specimen along the filter paper in one direction. Alternatively, the test device 5 may be configured such that the specimen spreads out in a circular pattern from the drop position, for example. In this case, the area spreading out from the spot at the drop position in the same shape may be designated as the first color-developing section or the second color-developing section.

In the above embodiments, the labeling substance that reacts with the target substance is not limited to gold colloidal particles modified with specific binding molecules. The labeling substance may be any substance capable of specific binding, such as fluorescent particles, Qdots (registered trademark), aptamers, etc. Fluorescent particles and Qdots develop red color when adsorbed onto gold colloidal particles. An aptamer is a molecule that specifically binds to a target molecule. Examples of aptamers include nucleic acid aptamers and peptide aptamers. In particular, peptide aptamers, which possess a structural feature of binding at two points, exhibit higher binding affinity compared to antibodies. Examples of peptide aptamer include binding enhanced fluorogenic (BEF) peptide, which emits fluorescence upon binding to the target.

In the above embodiments, the test apparatus device 4, connected to the user terminal 2 via the communication line 3, determines the amount of the target substance.

However, in implementations where the computer program P2 is installed on the user terminal 2, the user terminal 2 may also function as the test apparatus 4. In this case, the imaging device 26 provided on the user terminal 2 functions as the data acquisition device IG to acquire image data 20 including the test device 5 and the base sheet 6, while the control device 21 of the user terminal 2 functions as a determination device to determine the amount of the target substance from the image data 20. Alternatively, the test apparatus 4 may acquire image data 20 using a camera or other imaging device without using the user terminal 2 and perform automatic determination. In this case, the test results may be transmitted to the user terminal 2 via the communication line 3 or notified by mail or other means.

The present invention is not limited to the above embodiments, and it would be apparent that the respective embodiments may be appropriately modified or altered within the scope of the technical concept of the present invention. The technologies of each embodiment may be used in other embodiments as long as there are no technical discrepancies.

**Claims**

1. A test apparatus, comprising:

    a data acquisition device that acquires image data comprising:

    a color-developing section that reacts with a target substance in a specimen to develop color; and
    a reference section that displays a sample color used to determine color of the color-developing section and a reference color used to correct positioning-induced variations; and

    a determination device that determines an amount of the target substance based on color information of the color-developing section, color information of the sample color, and color information of the reference color, which are acquired from the image data.

2. The test apparatus according to claim 1, wherein the reference section comprises:

    a chart section comprising a color sample that displays the sample color to be compared with color of the color-developing section; and
    a reference display section that displays the reference color for estimating corrected color information that compensates for positioning-induced variations for at least one of the color-developing section and the color sample.

3. The test apparatus according to claim 2, wherein the determination device determines the amount of the target substance, estimating the corrected color information for at least one of the color-developing section and the color sample based on gradient information calculated from color information of the reference display section.

4. The test apparatus according to claim 2 or 3, wherein the chart section comprises a plurality of the color samples with different gradations, and wherein the reference color is the same as an intermediate color out of the plurality of the color samples.

5. The test apparatus according to any one of claims 2 to 4, wherein the chart section serves as the reference display section through an arrangement and coloring settings of the chart section.

6. The test apparatus according to any one of claims 1 to 5, wherein the color-developing section is disposed on a test device that receives the specimen.

7. The test apparatus according to claim 6, wherein the test device is any of an antibody test device, an antigen test device, or a biomarker test device.

8. The test apparatus according to claim 6, wherein the reference section is provided on a base sheet that comprises a placement area on which the test device is to be placed.

9. A test system, comprising:

a data acquisition device that acquires image data comprising:

a color-developing section that reacts with a target substance in a specimen to develop color; and
a reference section that displays a sample color used to determine color of the color-developing section and a reference color used to correct positioning-induced variations; and

a determination device that determines an amount of the target substance based on color information of the color-developing section, color information of the sample color, and color information of the reference color, which are acquired from the image data.

10. The test system according to claim 9, further comprising a user terminal that transmits the image data to the data acquisition device.

11. A test method, comprising:

acquiring image data comprising:

a color-developing section that reacts with a target substance in a specimen to develop color;
a reference section that displays a sample color used to determine color of the color-developing section and a reference color used to correct positioning-induced variations; and

determining an amount of the target substance based on color information of the color-developing section, color information of the sample color, and color information of the reference color, which are acquired from the image data.

12. A non-transitory storage medium storing a program which causes a control device to:

acquire image data comprising:

a color-developing section that reacts with a target substance in a specimen to develop color;
a reference section that displays a sample color used to determine color of the color-developing section and a reference color used to correct positioning-induced variations; and

determine an amount of the target substance based on color information of the color-developing section, color information of the sample color, and color information of the reference color, which are acquired from the image data.

FIG. 1

FIG. 2

FIG. 3

FIG. 4

FIG. 5

FIG. 6

FIG. 7

Start

S1

Determine color information and position of
each section within image data

S2

Calculate gradient information of reference
display section

S3

Calculate gradient between color samples
and first color-developing section

S4

Calculate estimated values of color
information of color samples at the position
of the first color-developing section

S5

Determine target substance amount from
estimated values of color information of
color samples and color information of first
color-developing section

End

# FIG. 8

FIG. 9

User Terminal

Test Device

Start

Start

S11

Acquire Image Data

S12

Transmit Image Data

Image Data etc.

S21

Receive Image Data

S22 (S1)

Determine color information and position
of each section within image data

S23

Determine existence of
target substance

S24 (S2~S5)

Determine target substance amount

S25

Transmit test results

S16

Test Results etc.

Receive Test Result

S17

Display Test Result

End

End

FIG. 10

FIG. 11

FIG. 12

EP 4 722 721 A1

FIG. 13

26

20 (10)

62
(69)

B    55
(TL)    5    53    56
(CL)

6

L2

AR2

V1

BC

AR1

SC

A    54    L1    52    51    C

BC

AR2

67

Y

X

FIG. 14

## INTERNATIONAL SEARCH REPORT

| International application No. |
|---|
| **PCT/JP2023/019667** |

**A. CLASSIFICATION OF SUBJECT MATTER**

*G01N 33/543*(2006.01)i
FI: G01N33/543 531

According to International Patent Classification (IPC) or to both national classification and IPC

**B. FIELDS SEARCHED**

Minimum documentation searched (classification system followed by classification symbols)

G01N33/52; G01N33/543; G01N21/78; H04N1/46-1/64; G01T1/00; G06T7/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2007-101482 A (MATSUSHITA ELECTRIC INDUSTRIAL CO., LTD.) 19 April 2007 (2007-04-19) claims, paragraphs [0032]-[0034], [0040], [0041], [0061]-[0069], second embodiment, fig. 1, 3, 4, 6 | 1-2, 6-12 |
| Y | | 3 |
| Y | JP 10-84550 A (FUJI PHOTO FILM CO., LTD.) 31 March 1998 (1998-03-31) claim 1, paragraph [0011] | 3 |
| A | JP 2007-81580 A (CANON KABUSHIKI KAISHA) 29 March 2007 (2007-03-29) entire text, all drawings | 1-12 |
| A | JP 2013-223048 A (CANON KABUSHIKI KAISHA) 28 October 2013 (2013-10-28) entire text, all drawings | 1-12 |
| A | JP 2023-49300 A (REPUBLIC WATER LLC) 10 April 2023 (2023-04-10) entire text, all drawings | 1-12 |
| A | JP 2015-533211 A (SIDHANT, Jena) 19 November 2015 (2015-11-19) entire text, all drawings | 1-12 |

✓ Further documents are listed in the continuation of Box C.      ✓ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **27 June 2023** | **11 July 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

**EP 4 722 721 A1**

## INTERNATIONAL SEARCH REPORT

| International application No. |
|---|
| **PCT/JP2023/019667** |

**C.    DOCUMENTS CONSIDERED TO BE RELEVANT**

| Category* | Citation of document, with indication, where appropriate, of the relevant passages | Relevant to claim No. |
|---|---|---|
| A | JP 2017-125840 A (TOPPAN PRINTING CO., LTD.) 20 July 2017 (2017-07-20)<br>entire text, all drawings | 1-12 |
| A | JP 2020-168602 A (JAPAN ORGANO CO., LTD.) 15 October 2020 (2020-10-15)<br>entire text, all drawings | 1-12 |
| A | JP 2018-155490 A (DAINIPPON PRINTING CO., LTD.) 04 October 2018 (2018-10-04)<br>entire text, all drawings | 1-12 |
| A | US 2015/0211987 A1 (SCANADU INCORPORATED) 30 July 2015 (2015-07-30)<br>entire text, all drawings | 1-12 |
| A | JP 2017-175568 A (FUJI XEROX CO., LTD.) 28 September 2017 (2017-09-28)<br>entire text, all drawings | 1-12 |
| A | KR 10-2022-0159601 A (FITPET CO., LTD.) 05 December 2022 (2022-12-05)<br>entire text, all drawings | 1-12 |
| A | US 6654048 B1 (MEAT & LIVESTOCK AUSTRALIA LIMITED) 25 November 2003<br>(2003-11-25)<br>entire text, all drawings | 1-12 |

Form PCT/ISA/210 (second sheet) (January 2015)

**INTERNATIONAL SEARCH REPORT**
Information on patent family members

International application No.

**PCT/JP2023/019667**

| Patent document cited in search report | | | Publication date (day/month/year) | Patent family member(s) | | | Publication date (day/month/year) |
|---|---|---|---|---|---|---|---|
| JP | 2007-101482 | A | 19 April 2007 | (Family: none) | | | |
| JP | 10-84550 | A | 31 March 1998 | US | 6211973 | B1 | |
| | | | | claim 1, column 3, line 54 to column 4, line 13 | | | |
| JP | 2007-81580 | A | 29 March 2007 | US | 2007/0058223 | A1 | |
| | | | | whole document | | | |
| | | | | EP | 1763219 | A1 | |
| | | | | CN | 1933545 | A | |
| JP | 2013-223048 | A | 28 October 2013 | (Family: none) | | | |
| JP | 2023-49300 | A | 10 April 2023 | (Family: none) | | | |
| JP | 2015-533211 | A | 19 November 2015 | US | 2014/0072189 | A1 | |
| | | | | whole document | | | |
| | | | | WO | 2014/037820 | A2 | |
| | | | | EP | 2893477 | A2 | |
| | | | | CN | 104812292 | A | |
| | | | | BR | 112015004900 | A2 | |
| JP | 2017-125840 | A | 20 July 2017 | (Family: none) | | | |
| JP | 2020-168602 | A | 15 October 2020 | (Family: none) | | | |
| JP | 2018-155490 | A | 04 October 2018 | (Family: none) | | | |
| US | 2015/0211987 | A1 | 30 July 2015 | WO | 2014/025415 | A2 | |
| | | | | whole document | | | |
| | | | | EP | 2883037 | A2 | |
| | | | | CN | 104969068 | A | |
| | | | | HK | 1215732 | A1 | |
| JP | 2017-175568 | A | 28 September 2017 | US | 2017/0278257 | A1 | |
| | | | | whole document | | | |
| KR | 10-2022-0159601 | A | 05 December 2022 | WO | 2022/250335 | A1 | |
| | | | | whole document | | | |
| US | 6654048 | B1 | 25 November 2003 | WO | 1998/039627 | A2 | |
| | | | | whole document | | | |
| | | | | EP | 1314308 | A2 | |
| | | | | AU | PO542897 | A | |
| | | | | ZA | 981760 | A | |
| | | | | AU | 6082798 | A | |
| | | | | BR | 9815462 | A | |
| | | | | NZ | 337986 | A | |
| | | | | CA | 2283063 | A1 | |
| | | | | MX | 9907667 | A | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2022119435 A
- JP 2016102790 A
- JP H10165375 A

- JP 2016161301 A
- JP 2017515106 A